Europäisches Patentamt

(19) European Patent Office

Office européen des brevets

(11) **EP 0 488 928 B1**

(12) **EUROPEAN PATENT SPECIFICATION**

(45) Date of publication and mention
of the grant of the patent:
**25.08.1999 Bulletin 1999/34**

(51) Int. Cl.$^6$: **A61K 35/78**, A61K 31/045

(21) Application number: **91500137.4**

(22) Date of filing: **29.11.1991**

(54) **Pharmaceutical formulations containing a mixture of higher primary aliphatic alcohols in the treatment of hypercholesterolaemia and hyperlypoproteinaemia type II and stimulation of sexual behavior in animals and humans**

Arzneimittel enthaltend Mischungen von höheren primären aliphatischen Alkoholen zur Behandlung von Hypercholesterolämie und Hyperlipoproteinämie Typ II und zur Stimulation des sexuellen Verhaltens bei Tieren und Menschen

Médicament contenant des mélanges d'alcools aliphatiques primaires à chaines longues, pour traiter l'hypercholésterolémie et l'hyperlipoprotéinémie du type II et pour stimuler le comportement sexuel des animaux et des hommes

(84) Designated Contracting States:
**AT BE CH DE DK ES FR GB GR IT LI NL SE**

(30) Priority: **30.11.1990 CU 18590**

(43) Date of publication of application:
**03.06.1992 Bulletin 1992/23**

(73) Proprietor:
**CENTRO NACIONAL DE INVESTIGACIONES CIENTIFICAS
La Habana (CU)**

(72) Inventors:
• **Laguna Granja, Abilio**
**La Habana (CU)**
• **Magraner Hernandez, Juan**
**La Habana (CU)**
• **Ramos Lezcano, Rubén Pablo**
**La Habana (CU)**
• **Urribari Hernandez, Evangelina**
**La Habana (CU)**
• **Perdomo Naranjo, Urbano Gregorio**
**La Habana (CU)**
• **Carvajal Fernández, Daysi**
**La Habana (CU)**
• **Arruzazabala, Maria de Lourdes**
**La Habana (CU)**
• **Martinez Rojas, José Manuel**
**La Habana (CU)**
• **Lorenzo Otero, Margarita Juana**
**La Habana (CU)**

• **Montejo Loret De Mola, Liliana Magdalena**
**La Habana (CU)**
• **Mas Ferreiro, Rosa**
**La Habana (CU)**

(74) Representative: **Gil-Vega, Victor**
**Estébanez Calderon, 3 - 5B
28020 Madrid (ES)**

(56) References cited:
**US-A- 4 714 791**

• **WORLD PATENTS INDEX LATEST Derwent Publications Ltd., London, GB; AN 86-282497 & JP-A-61 207 321**
• **CHEMICAL ABSTRACTS, vol. 106, no. 17, Columbus, Ohio, US; abstract no. 137413P, FUKUDA ET AL.: 'Effects of sugarcane wax on serum and liver lipids in rats'**
• **WORLD PATENTS INDEX LATEST Week 8724, Derwent Publications Ltd., London, GB; AN 87-167050 & JP-A-62 099 323 (HAGIWARA Y.) 8 May 1987**
• **J.NUTR.VITAMINOL. vol. 30, no. 6, 1984, pages 553 - 559; HIROKO SHO ET AL.: 'Effects of Okinawan Sugar Cane Wax and Fatty Alcohol on Serum and Liver Lipids in the Rat'**
• **J.NUTR.SCI.VITAMINOL. vol. 29, no. 3, 1983, pages 313 - 322; HIROKO SHO ET AL.: 'Separation and Partial Purification of Wax and Fatty Alcohol from Okinawan Sugar Cane Rind Lipids'**

## Description

The present invention is mainly related to the pharmaceutical industry and, in particular, to the effective formulation of hypercholesterolaemia and reduction of LDL levels. It has also proven to be an important stimulator of sexual behaviour. For the preparation of these formulations a mixture of saturated primary alcohols having a straight chain ranging from 24 to 34 carbon atoms in specific relation to each other is used.

The first objective of this invention is to use a mixture of higher primary aliphatic alcohols obtained from sugar cane wax as an active compound.

The second objective is the formulation of pharmaceutical components which use as active compound is the mixture of higher primary aliphatic alcohols with important pharmacological properties to be administered both orally and parenterally.

Drugs with specific pharmacological properties based on the use of a mixture of higher primary fatty alcohols as an active compound are not known.

Formulations used as a nutritional suplementation based on mixtures of higher primary fatty alcohols have been obtained in recent years, especially in an attempt to find the ergogenic properties reported first in the case of wheat gem oil and later in octacosanol.

In 1985, Sanyu Shoji **(JP-A-60049775)** reported the components of a dietary supplementation containig *Eleutherococus senticosus* extract, pangamic acid and octacosanol, the latter accounting for 30 to 60% of the dietary supplementation which increases the general activity of internal organs such as the heart, the liver and the kidneys. The Japanese patent JP 85-119514 (Pub. No. 62-089637) is associated with the obtention of higher primary aliphatic alcohols from sugar cane wax. It also reports that octacosanol has two main effects, namely, to increase physical strength and to restore the damaged nervous cells as well as to lower blood pressure and improve muscular functions including the myocardium.

In the Japanese patent JP-A-61 207 321 a hair tonic is described which contains as active agent a mixture of higher aliphatic alcohols having 22 to 34 carbon atoms.

Patent **JP-A-62224258** includes the formulation of a nutritional food based on the addition of different types of lipids such as palmitic acid, docohexanoic acid, eicosapentanoic acid, linolenic acid, lecithins, vitamin E and octacosanol as dietary supplementation. This dietary supplementation was administered to rats which were previously fed with high cholesterol diet. The accumulation of blood cholesterol was eliminated after two weeks treatment. The hypocholesterolaemic properties of thid product derive mainly from the presence of fatty acids and lecithins.

Nogushi *et al*, patent **JP-A-63116645**, report the formulation of a candy containing octacosanol used as an ergogenic supplementation at a 1:89 ratio in diets of rats subject to physical activity. This physical activity lasted two weeks and, afterwards, it was found that glycogen levels in muscles and liver had decreased in relation to normal-diet animals.

According to patent **JP-A-5410539**, another property reported in the case of octacosanol is that of increasing the presence of sex hormones. Similarly, it states that octacosanol regulates hormonal functions.

Many comercial products with ergogenic properties based on mixtures of higher primary alcohols are also known, as for example the so-called Endurol which contains 33% octacosanol. 35% triacontanol, 20% tetracosanol and 12% hexacosanol. The Viobin company has marketed an ergogenic tonic based on the same higher primary alcohols, though in different amounts. It has been reported that this tonic contains 33% octacosanol, 41.6% triacontanol, 16.6% tetracosanol and 8.3% hexacosanol.

In Japanese patents **JP-A-60049775** and **JP-A-62224258** reference is made to 26-30 carbon atoms saturated alcohols generally presenting the same physiological properties. However, previous studies have shown that this is not so. For example, Jones R.L. *et al*, Plant Physiol.(1995) 79, 357-64 in experiments carried out with Chalydomonas, proved that TRIA stimulates growth and assimilation of $CO_2$ by photosynthesis while octacosanol inhibits TRIA effects during the $CO_2$ assimilation process. In similar studies, Ries S.K. *et al*, Planta (1987) 172, 79-87, found that octacosanol inhibits triacontanol plant growth promotion properties. Subsequent in vitro experiments by Lesniak A.P. *et al*, Physiol.Plant. (1989) 75, 75-80, indicate that triacontanol increasingly stimulates ATPase activity in Barley vesicle membrane (Hordeum vulgare) while octacosanol did not.

There is also another study (Borg D. *et al*, J. Neurology (1987) 33, 475-79) regarding the effect of long chain alcohols in neural growth which reveals that hexacosanol is powerful neural growth stimulator. Alcohols of 16, 20, 22, 24, 28 and 30 carbon atoms had no significant effects.

Shoh H. *et al*, J. Nutr.Sci. Vitaminol (1984) 30, 553-9, studied the effects of Okinawa sugar cane wax on serum and liver lipid levels in rats subjected to diets containing 0.5% of this sugar cane wax and found a significant reduction of cholesterol levels in these rats' serum and liver. No significant variations in cholesterol levels were found when using fatty alcohols from this wax in these diet-fed rats. **Shumira S. *et al*, Nutritional Report Int. (1987) 36, 1029-38** studied the octacosanol effects in mice subject to physical activity and fed with octacosanol enriched diets. These assays showed that octacosanol increases physical strength. At the same time, they found a significant decrease of neutral lipids and cholesterol levels in the liver without modifications in the phospholipids content. Nevertheless, reduction of serum cholesterol and lipoprotein levels were not observed. There are different commercial drugs used in the treatment of hypercholesterolaemic patients which lower cholesterol levels by 30% but they all provoke a number af negative effects. For example, Lopid used in the treatment of hypercholesterolaemia, reduces LDL-cholesterol by 5.4 and 6.9% but it decreases sexual capacity in some patients, causes skin rash, headaches and blurred vision. Likewise, it should not be administered to patients with renal failure. Probucol, which causes a 10% mean decrease of cholesterol level and from 10 to 15% LDL-cholesterol, produces gastrointestinal disturbances, nauseas, abdominal pain, diarrheas and flatulence in 10% of the population, as well as a considerable variation in the electrocardiogram.

Another product used in the treatment of hypercholersterolaemic patients is Cholestyramine. This drug is highly efficient as hypercholesterolaemic since it lowers serum cholesterol levels by 39% and LDL by 30%. However, this product has some disadvantages since the dosage required is relatively high (16-20 g daily), causing constipation and interacting with other drugs like Digitoxin.

Mevacor has been broadly used in recent years due to its rapid and lowering effects in cholesterol levels, namely 32% reduction of cholesterol and 39% of LDL. The disadvantage of this product is that it provokes a number of adverse effects including gastrointestinal disturbances, headaches, subcutaneous rash, pruritus, and severe muscular lesions in sensitive patients resulting in myolisis, and testicular atrophy. It increases creatinekinase and transaminase since hepatic tumors in laboratory animal have been reported. Zocor is a Mevacor by-product causing mild adverse effects. Reportedly, constipation, flatulence, nausea, headache, fatigue, subcutaneous rash and myopathies affecting creatinekinase are some of the disadvantages of this product. One of the objectives of this invention is to use the mixture of saturated primary fatty alcohols with 24 to 32 carbon atoms as a component of the pharmaceutical formulation. The mixture formulation has been determined by using gas chromatography in a capillary column, the formulation of the mixture is derivatized when these alcohols are dissolved in pyridine and are silonized using N-methyl-N-TMS-trifluoracetamide (MSTFA). Table I shows the qualitative and quantitative results of the mixture.

TABLE I

| Qualitative and quantitative formulation of the mixture of primary fatty alcohols used | |
| --- | --- |
| Component | Proportion in the mixture (%) |
| tetracosanol | 0.5- 5.0 |
| hexacosanol | 5.0-15.0 |
| heptacosanol | 0.5- 5.0 |
| octacosanol | 55.0-80.0 |
| nonacosanol | 0.5- 3.0 |
| triacontanol | 6.0-20.0 |
| dotriacontanol | 1.0-10.0 |
| tetratriacontanol | 0.0- 2.5 |

The aim of this invention is that the mixture of saturated primary alcohols, **which is used as** an active compound in pharmaceutical formulations, is made up by saturated primary alcohols having a long chain ranging from 24-34 carbon atoms. This alcohol mixture is obtained from sugar cane wax, a semi-crystalline, off-white color solid with a 78.0 - 82.5°C fusion temperature.

This mixture is obtained from different types of waxes through a homogeneous stage saponification process and its subsequent extraction with organic solvents.

**In the United States Patent US-A-4 714 791 a method for recovering primary normal aliphatic higher alcohols having 20 to 36 carbon atoms from sugarcanes or sugarcane products is disclosed, using as extractant a fluid in a subcritical or supercritical state. In a preferred embodyment in the US patent the use of $CO_2$ for the extraction is described.**

The remarkable aspect of this finding is that the mixture of alcohols causes a marked reduction of cholesterol and other low density serum lipoproteins (LDL) which is of great importance in the control of risk factors. The mean reduction of cholesterol and LDL in this experiment is 12 and 13% respectively. Furthermore, in comparative studies on the effects of octacosanol and the mixture of alcohols in bloos lipid levels, we have found that the alcohol mixture -**used** as an active compound- lowers serum cholesterol levels, changes plasmatic lipoproteins patterns, increases high density lipoproteins (HDL) and lowers LDL thus causing an increase in the HDL/LDL ratio while with the use of octacosanol this effect was not observed.

The aforementioned findings are amazing if we take into account that **Sho H.** *et al*, **J. Nutr. Sci. Vitaminol, (1984) 30, 553-9** were not able to obtain significant results on serum lipid levels with the use of fatty alcohols from the Okinawa sugar cane.

**The content of the mixture of alcohols in the daily dosage** suitable for hypercholesterolaemia treatment is from 1 to 50 mg. The most adequate administration way is orally through coated tablets of granules although this drug can be administered parenterally.

The pharmaceutical formulation single dose to be orally administered contains, mostly as an active substance, from 0.5 to 5 wt% of the alcohol mixture. This dosage is obtained by mixing the active substance with different excipients in the pharmaceutical formulation either as agglutinants, disintegrators, lubricants, sliders or just fillers.

The drug proposed by this invention is relatively innocuous according to the results obtained in acute, subacute, subchronic and chronic toxicity assays carried out in rodents as well as in chronic studies in monkeys. They do not reveal any teratogenic activity in rats or rabbits, nor do they have a mutagenic potential.

No side-effects have been detected in patients treated with the drug resulting from this invention. However, when administered, there is an increase of sexual activity of laboratory animals, whose mechanism of action is only based on the increase of libido, this effect having no relation with the level of sexual hormone mainly involved in the control of such behaviour. There is a number of patients who have also referred to this effect.

The objetive of this invention will be further on detailed and will refer to the examples which will not be restricted to the scope of said invention.

**Example 1**

1 kg of raw sugar cane wax is taken to which a homogeneous phase saponification process is applied followed by the extraction of primary fatty alcohols using the adequate organic solvents. 105 g of said alcohols mixture were obtained. The fusion temperature of the alcohol mixture ranges from 78.0 to 82.0°C and the purity of the mixture is 92.98%.

**Example 2**

Out of 2.5 kg of refined sugar cane wax, following the method used in the aforementioned example, 438.6 g of alcohol mixture were obtained in this case accounting for a 95.91 % purity and the fusion temperature ranged from 79.5 to 82°C.

For analysis of alcohols through gas chromatography in a capillary column of fused silica, these alcohols are derivatized by using N methyl-N-TMS-trifluoroacetamide (MSTFA) dissolved in pyridine. Table II shows the results of qualitative and quantitative analysis in both examples.

TABLE II

| Qualitative and quantitative composition of the mixture of higher aliphatic alcohols obtained | | |
|---|---|---|
| Identified component | Percentage of alcohols | |
| | In example 1 | In example 2 |
| tetracosanol | 2.08 | 2.81 |
| hexacosanol | 9.68 | 7.57 |
| heptacosanol | 3.16 | 2.81 |
| octacosanol | 68.30 | 73.99 |
| nonacosanol | 0.44 | 0.85 |
| triacontanol | 8.17 | 6.41 |
| dotriacontanol | 1.05 | 1.35 |
| tetratriacontanol | 0.10 | 0.12 |

TABLE II (continued)

| Qualitative and quantitative composition of the mixture of higher aliphatic alcohols obtained | | |
|---|---|---|
| Identified component | Percentage of alcohols | |
| | In example 1 | In example 2 |
| Total | 92.98 | 95.91 |

## Example 3

From the mixture of higher alcohols obtained in Example 2, 5mg tablets were made from the alcohol mixture containing the components listed in Table III.

TABLE III

| Pharmaceutical formulation of the 5 mg tablets | |
|---|---|
| Component | % in tablet |
| Mixture of higher aliphatic alcohols | 4.5 |
| Lactose | 75.0 |
| Corn starch | 15.0 |
| Gelatin | 1.5 |
| Sodium croscarmelose | 1.0 |
| Talc | 2.0 |
| Magnesium estearate | 1.0 |

## Example 4

The tablet formulation, **prepared using 5 mg of the reported mixture of alcohols**, is administered to a group of 16 patients affected with hyperlypoproteinaemia type II (according to Frederickson's classification, **Frederickson D.S. et al, N. England J. Med. (1967) 276, 34, 94, 148, 215, 273**). Before these patients were included in the assay, they were subjected to a 4 weeks diet and only those who still had a high LDL were treated. The diet element was continued throughout the study and the research carried out on the main lipid-lowering drugs available. Treatment was continued for 8 weeks, whereby each patient was administered a daily tablet with the formulation described in the previous example. Laboratory assays were conducted after 4 and 8 weeks, and parameters in Tables IV and V were evaluated.

TABLE IV

| Effects of the formulation on serum-triglycerides and cholesterol levels | | | | | | |
|---|---|---|---|---|---|---|
| | Cholesterol (mmol/L) | | | Triglycerides (mmol/L) | | |
| | (weeks) | | | | | |
| Patient | 0 | 4 | 8 | 0 | 4 | 8 |
| 1 | 7.4 | 7.0 | 6.48 | 1.89 | 1.25 | 1.47 |
| 2 | 7.7 | 6.96 | 5.67 | 3.16 | 2.28 | 0.74 |
| 3 | 9.28 | 7.73 | - | 2.88 | 1.40 | - |
| 4 | 10.88 | 11.31 | 10.03 | 2.29 | 2.44 | 2.02 |
| 5 | 6.18 | 4.69 | 5.41 | 3.49 | 3.42 | 2.40 |
| 6 | 12.13 | 10.62 | 10.41 | 2.60 | 1.73 | 1.90 |

TABLE IV (continued)

| Effects of the formulation on serum-triglycerides and cholesterol levels | | | | | | |
|---|---|---|---|---|---|---|
| | Cholesterol (mmol/L) | | | Triglycerides (mmol/L) | | |
| | (weeks) | | | | | |
| Patient | 0 | 4 | 8 | 0 | 4 | 8 |
| 7 | 10.58 | - | 9.27 | 2.40 | - | 2.67 |
| 8 | 6.45 | 6.84 | 5.85 | 2.08 | 3.39 | 1.83 |
| 9 | 7.20 | 7.13 | - | 2.36 | 2.01 | - |
| 10 | 8.05 | 7.26 | 6.60 | 2.61 | 1.47 | 3.73 |
| 11 | 7.66 | 9.03 | 7.16 | 2.57 | 3.27 | 2.63 |
| 12 | 8.08 | 6.33 | 7.10 | 6.22 | 3.31 | 3.79 |
| 13 | 7.98 | 8.99 | 7.20 | 0.76 | 1.64 | 0.91 |
| 14 | 8.43 | 6.40 | 8.16 | 1.78 | 2.35 | 3.98 |
| 15 | 8.27 | 6.52 | 6.97 | 5.34 | 5.18 | - |
| 16 | 7.98 | 8.59 | 7.39 | 1.30 | 0.78 | 0.95 |
| $\overline{X}$ | 8.38 | 7.69 | 7.42 | 2.73 | 2.33 | 2.32 |

Table 4 shows that after 8 weeks there is a reduction of serum cholesterol and triglycerides in patients treated with this formulation. In the case of cholesterol, the reduction was statistically meaningful ($p < 0.05$ Wilcoxon), whereas no significant level was reached in triglycerides. Mean reduction in serum cholesterol levels was 12.2%.

The analysis of the results revealed that treatment resulted in a significant decrease of cholesterol and low density lipoproteins. Treatment was 100% effective since total cholesterol level in all patients was lower than at the beginning and the same applies for LDL when initial measurements were made. The percentage of LDL reduction was 13%.

TABLE V

| Effect of the formulation on low density lipoproteins (LDL) and very low density lipoproteins (VLDL) in plasma | | | | | | |
|---|---|---|---|---|---|---|
| | Lipoproteins analyzed (mmol/L) | | | | | |
| | LDL | | | VLDL | | |
| Patient | 0 | 4 | 8 | 0 | 4 | 8 |
| | (weeks) | | | | | |
| 1 | 5.25 | 5.02 | 4.75 | 0.86 | 0.57 | 0.67 |
| 2 | 4.91 | 4.92 | - | 1.44 | 1.04 | 0.33 |
| 3 | 6.77 | 6.20 | - | 1.31 | 0.64 | - |
| 4 | 8.45 | 9.01 | 8.11 | 1.04 | 1.11 | 0.92 |
| 5 | 3.48 | - | 3.01 | 1.59 | 1.10 | 1.55 |
| 6 | 9.95 | 8.85 | 8.66 | 1.18 | 0.79 | 0.86 |
| 7 | 7.70 | - | 6.31 | 1.09 | - | 1.21 |
| 8 | 4.42 | 4.27 | - | 0.95 | 1.44 | 0.83 |
| 9 | 4.72 | 5.24 | - | 1.07 | 0.91 | - |
| 10 | 6.18 | 4.64 | 4.14 | 1.19 | 0.67 | 1.64 |
| 11 | 5.47 | 6.34 | 4.94 | 1.17 | 1.49 | 1.20 |

TABLE V (continued)

| Effect of the formulation on low density lipoproteins (LDL) and very low density lipoproteins (VLDL) in plasma | | | | | | |
|---|---|---|---|---|---|---|
| | Lipoproteins analyzed (mmol/L) | | | | | |
| | LDL | | | VLDL | | |
| Patient | 0 | 4 | 8 | 0 | 4 | 8 |
| | (weeks) | | | | | |
| 12 | 4.04 | 3.66 | 3.95 | 2.83 | 1.50 | 1.72 |
| 13 | 5.25 | 6.04 | 4.72 | 0.35 | 0.75 | 0.41 |
| 14 | 6.31 | 4.33 | 5.07 | 0.81 | 1.07 | 1.81 |
| 15 | 4.78 | 3.25 | - | 2.61 | 2.35 | - |
| 16 | 5.51 | 6.16 | 5.28 | 0.59 | 0.35 | - |
| $\overline{X}$ | 5.82 | 5.56 | 5.33 | 1.26 | 1.06 | 1.05 |

**Example 5**

A group of patients with hypercholesterolaemia were treated with 15 mg tablets of the formulation for 3 months. Results are shown in Table VI. In this example, there is also a cholesterol and triglycerides reduction in all treated patients.

Total cholesterol reduction amounted to 17.32% and serum triglycerides to 16.3% after and before treatment.

**Example 6**

Formulations containing between 0.5 and 5 mg of the alcohol mixture per kilogram of bodyweight were orally administrated to a group of New Zealand male rabbits during a month. The vehicle was administered to a control group included in the study. Blood samples were taken every 15 days to determine the parameters of lipidic metabolism. Results are shown in tables VII and VIII.

TABLE VI

| Effect of the formulation on cholesterol and serum triglycerides levels in a group of patients | | | | | | | | |
|---|---|---|---|---|---|---|---|---|
| | Cholesterol | | | | Triglycerides | | | |
| | (mmol/L) | | | | | | | |
| Patient | 0 | 30 | 60 | 90 | 0 | 30 | 60 | 90 |
| | (days) | | | | | | | |
| 1 | 7.5 | 6.8 | 6.5 | 5.9 | 1.78 | 1.65 | 1.37 | 1.42 |
| 2 | 7.19 | 7.0 | 6.84 | 6.63 | 3.15 | 2.66 | 2.34 | 2.05 |
| 3 | 10.3 | 9.5 | 8.8 | 8.6 | 2.15 | 1.88 | 1.63 | 1.58 |
| 4 | 6.3 | 6.2 | 6.0 | 5.5 | 3.51 | 3.02 | 2.73 | 2.22 |
| 5 | 7.0 | 6.5 | 5.5 | 5.3 | 2.78 | 2.70 | 2.35 | 2.00 |
| 6 | 8.6 | 8.3 | 7.6 | 7.7 | 1.80 | 1.65 | 1.53 | 1.54 |
| 7 | 9.2 | 8.5 | 7.6 | 7.0 | 2.20 | 1.99 | 1.76 | 1.71 |
| 8 | 8.6 | 8.8 | 8.0 | 6.8 | 3.42 | 3.15 | 2.70 | 2.52 |
| 9 | 10.5 | 9.9 | 9.3 | 9.0 | 2.87 | 3.01 | 2.57 | 2.42 |
| 10 | 11.4 | 11.3 | 9.9 | 9.2 | 2.56 | 2.68 | 2.49 | 2.35 |

TABLE VI (continued)

| Effect of the formulation on cholesterol and serum triglycerides levels in a group of patients | | | | | | | |
|---|---|---|---|---|---|---|---|
| | Cholesterol | | | | Triglycerides | | |
| | (mmol/L) | | | | | | |
| Patient | 0 | 30 | 60 | 90 | 0 | 30 | 60 | 90 |
| | (days) | | | | | | |
| X̄ | 8.66 | 8.28 | 7.6 | 7.16 | 2.62 | 2.43 | 2.11 | 2.09 |

TABLE VII

| Effect of the formulation on cholesterol and triglycerides levels in rabbits | | | | | | | |
|---|---|---|---|---|---|---|---|
| Animal | Dosage (mg/kg) | Cholesterol | | | Triglycerides | | |
| | | (mmol/L) | | | | | |
| | | 0 | 15 | 30 | 0 | 15 | 30 |
| | | (days) | | | | | |
| 1 | 0.0 | 3.25 | 2.60 | 4.40 | 1.23 | 1.09 | 2.12 |
| 2 | | 2.86 | 3.54 | 5.74 | 0.83 | 1.01 | 0.84 |
| 3 | | 1.54 | 2.72 | 3.24 | 1.73 | 1.40 | 1.43 |
| 4 | | 2.25 | 1.77 | 1.52 | 1.59 | 1.19 | 1.53 |
| 5 | | 1.90 | 2.94 | 2.47 | 0.96 | 1.34 | 1.01 |
| 6 | | 1.81 | 2.48 | 1.82 | 0.66 | 1.11 | 0.84 |
| 7 | | 2.00 | 3.09 | 2.26 | 2.11 | 3.29 | 1.27 |
| X̄ | | 2.17 | 2.55 | 2.74 | 1.45 | 1.40 | 1.11 |
| 8 | 0.5 | 3.86 | 4.42 | 5.56 | 1.41 | 1.53 | 1.42 |
| 9 | | 1.96 | 2.69 | 2.00 | 1.26 | 1.01 | 0.59 |
| 10 | | 2.74 | 2.01 | 2.03 | 0.64 | 0.97 | 0.49 |
| 11 | | 2.38 | 2.61 | 2.47 | 1.32 | 1.42 | 0.48 |
| 12 | | 1.14 | 1.07 | 1.23 | 0.93 | 1.62 | 1.22 |
| 13 | | 1.38 | 2.13 | 2.54 | 1.03 | 1.01 | 0.71 |
| 14 | | 2.83 | 2.02 | 1.46 | 2.26 | 1.61 | 1.16 |
| X̄ | | 2.32 | 2.42 | 2.47 | 1.26 | 1.31 | 0.86 |
| 15 | 5.0 | 1.48 | 1.67 | 1.76 | 0.93 | 0.98 | 0.69 |
| 16 | | 2.42 | 1.93 | 1.94 | 0.57 | 0.89 | 0.62 |
| 17 | | 1.86 | 1.89 | 1.83 | 0.79 | 1.30 | 0.69 |
| 18 | | 2.04 | 2.10 | 2.24 | 2.04 | 1.08 | 0.95 |
| 19 | | 1.96 | 1.67 | 1.53 | 0.88 | 0.68 | 0.63 |
| 20 | | 3.07 | 3.61 | 3.85 | 1.78 | 1.14 | 0.98 |
| 21 | | 5.53 | 3.02 | 2.04 | 3.28 | 1.38 | 0.64 |
| X̄ | | 2.62 | 2.27 | 2.17 | 1.46 | 1.06 | 0.74 |

A significant difference was found in the content of serum cholesterol an tryglycerides of rabbits treated with the formulation in a concentration of 5 mg of the mixture of aliphatic alcohols per kg of bodyweight by comparing its variation in time with that of the control group.

As can be observed in the Table, there is a significant reduction in VLDL with the **formulation in a concentration** of 5.0 mg of the alcohol mixture per kg when compared to the control group after 30 days. Other parameters are not significantly affected and a tendency is towards a reduced LDL, which is compatible with the results obtained for humans.

TABLE VIII

| Effect of the formulation on the lipoproteins in rabbit serum | | | | | | | | | | |
|---|---|---|---|---|---|---|---|---|---|---|
| Animal | Doses (mg/kg) | HDL | | | LDL | | | VLDL | | |
| | | 0 | 15 | 30 | 0 | 15 | 30 | 0 | 15 | 30 |
| | | (days) | | | | | | | | |
| 1 | 0 | 0.51 | 1.28 | 0.99 | 2.28 | 0.83 | 2.45 | 0.56 | 0.46 | 0.96 |
| 2 | | 1.17 | 1.49 | 1.33 | 1.31 | 1.60 | 4.03 | 0.38 | 0.45 | 0.38 |
| 3 | | 0.56 | 1.00 | 0.70 | 0.19 | 1.09 | 1.89 | 0.79 | 0.63 | 0.65 |
| 4 | | 0.65 | 0.77 | 0.62 | 0.51 | 0.46 | 0.21 | 1.09 | 0.54 | 0.69 |
| 5 | | 0.56 | 1.23 | 1.01 | 0.90 | 1.10 | 1.01 | 0.44 | 0.61 | 0.45 |
| 6 | | 0.94 | 1.32 | 0.95 | 0.57 | 0.66 | 0.49 | 0.30 | 0.50 | 0.38 |
| 7 | | 0.77 | 0.88 | 0.90 | 0.34 | 0.72 | 0.79 | 0.96 | 1.49 | 0.57 |
| $\overline{X}$ | | 0.74 | 1.09 | 0.89 | 0.83 | 0.78 | 1.30 | 0.70 | 0.63 | 0.51 |
| 8 | 0.5 | 0.81 | 1.07 | 0.82 | 2.41 | 2.66 | 4.10 | 0.64 | 0.69 | 0.64 |
| 9 | | 0.69 | 1.24 | 1.03 | 0.70 | 0.99 | 0.71 | 0.57 | 0.46 | 0.26 |
| 10 | | 1.25 | 1.02 | 0.79 | 1.18 | 0.55 | 1.02 | 0.31 | 0.44 | 0.22 |
| 11 | | 0.77 | 1.00 | 0.86 | 1.01 | 0.97 | 1.40 | 0.60 | 0.64 | 0.21 |
| 12 | | 0.56 | 0.43 | 0.49 | 0.16 | 0.10 | 0.19 | 0.42 | 0.73 | 0.35 |
| 13 | | 0.57 | 0.90 | 0.83 | 0.99 | 0.40 | 0.45 | 1.04 | 0.74 | 0.52 |
| 14 | | 0.82 | 0.88 | 0.49 | 0.33 | 0.77 | 1.39 | 0.48 | 0.46 | 0.32 |
| $\overline{X}$ | | 0.78 | 0.93 | 0.75 | 0.96 | 0.90 | 1.32 | 0.57 | 0.59 | 0.38 |
| 15 | 5.0 | 0.92 | 0.90 | 0.73 | 0.14 | 0.33 | 0.72 | 0.42 | 0.44 | 0.31 |
| 16 | | 0.83 | 1.11 | 0.73 | 1.33 | 0.42 | 1.39 | 0.26 | 0.40 | 0.28 |
| 17 | | 0.78 | 0.86 | 0.85 | 0.55 | 0.66 | 1.11 | 0.36 | 0.59 | 0.31 |
| 18 | | 0.56 | 0.95 | 0.70 | 0.72 | 0.44 | 0.67 | 0.93 | 0.49 | 0.43 |
| 19 | | 0.80 | 0.94 | 0.75 | 0.72 | 0.48 | 0.50 | 0.40 | 0.31 | 0.28 |
| 20 | | 0.56 | 1.32 | 1.19 | 2.41 | 1.77 | 2.22 | 0.81 | 0.52 | 0.44 |
| 21 | | 0.63 | 1.71 | 0.92 | 3.41 | 0.69 | 0.83 | 1.49 | 0.62 | 0.29 |
| $\overline{X}$ | | 0.72 | 1.11 | 0.84 | 1.33 | 0.67 | 1.06 | 0.66 | 0.48 | 0.33 |

**Example 7**

30 New Zealand rabbits having similar mean cholesterol values were distributed in three equal groups. One of them was taken as a control group, while the other two were given **formulations in a concentration** of 5 mg/kg of bodyweight of octacosanol and the alcohol mixture respectively. Blood samples were taken when treatment began, and 15 and 30 days later. Results are shown in Table IX. The study shows that 5 mg/kg of bodyweight treatment for a month tended to increase HDL while the HDL/LDL ratio decreased in relation to both the control group and the octacosanol group.

### TABLE IX

Effects of octacosanol and of the alcohol mixture on lipoproteins in rabbit serum

| Serum levels of HDL-C | | | Serum levels of LDL-C (mmol/L) | | | HDL/LDL ratio | |
|---|---|---|---|---|---|---|---|
| 0 | 15 | 30 | 0 | 15 | 30 | 0 | 30 |
| | | | | (days) | | | |

Octacosanol

| 0.59 ± 0.18 | 0.53 ± 0.18 | 0.95 ± 0.22 | 1.10 ± 0.52 | 0.88 ± 0,7 | 1.42 ± 0.52 | 0.61 ± 0.21 | 0.69 ± 0.23 |

Alcohol mixture

| 0.52 ± 0.23 | 0.63 ± 0.24' | 1.05 ± 0.17' | 1.04 ± 0.33 | 0.91 ± 0.39 | 1.24 ± 0.45' | 0.62 ± 0.26 | 0.99 ± 0.44* |

Control

| 0.54 ± 0.18 | 0.43 ± 0.12 | 0.93 ± 0.22 | 1.01 ± 0.5 | 0.91 ± 0.61 | 1.46 ± 0.44 | 0.55 ± 0.23 | 0.68 ± 0.17 |

'Is tendency and *$p < 0.05$ test t for non-paired series

## Example 8

Daily administration of the formula resulting from this invention causes an increase of sexual activity in male rats which is expressed in an significant increase in the quantified number of erections and mounts during the observation of copulations with estrogenized females. These results are shown in Table X.

### TABLE X

| Effect of oral administration of the formulation on the sexual behavior of male rats | | | | |
|---|---|---|---|---|
| Treatment (mg/kg) | Mounts | Erections | Animals mounting | Animals with erection |
| | | | (%) | |
| | $(\overline{X} \pm DE)$ | | | |
| Control | 8 ± 14 | 8 ± 13 | 50 | 66 |
| 0.5 | 21 ± 21* | 22 ± 21* | 75 | 75 |
| 5.0 | 24 ± 19** | 37 ± 44*** | 83* | 92* |
| 50 | 23 ± 11** | 23 ± 10*** | 100* | 100** |
| Note: Columns showing the number of mounts and erections were analyzed according to Mann Whitney U Tests and those showing percents were analyzed according to Fischer's Multiple Proportions Test | | | | |

*$p < 0.05$;
**$p < 0.01$;
***$p < 0.001$

Experiments aimed at determining the effect of suspending the treatment for certain periods of time corroborate the above contention, since there is no significant difference as to sexual activity between formulation administered rats and controls. An increase in sexual activity has been observed in rats of up to 44 weeks of age when compared to controls.

**Example 9**

The effects of the mixture of primary fatty alcohols on the sexual behaviour of male monkeys (**Macaca arctoides**) were studied after a daily dosage of the formulation (2.5 mg and 25 mg of active compound per kg of animal weight). The results are shown in Table XI.

TABLE XI

| Effects of oral administration of 2.5 and 25 mg/kg dosages on the sexual behavior of Macaca arctoides monkeys and the serum testosterone levels | | | | |
|---|---|---|---|---|
| Treatment (mg/kg) | Animal | Erections | Masturbations | Testosterone levels (nmol/L) |
| Control | 1 | 3 | 2 | 15 |
| | 2 | 2 | 2 | 13 |
| | 3 | 2 | 2 | 28 |
| | 4 | 0 | 0 | 26,3 |
| | 5 | 3 | 3 | 11,8 |
| | 6 | 0 | 0 | 21,4 |
| 2.5 | 1 | 21 | 20 | 22 |
| | 2 | 2 | 2 | 19 |
| | 3 | 3 | 2 | 22 |
| | 4 | 20 | 15 | 39 |
| 25 | 1 | 16 | 5 | 19,6 |
| | 2 | 7 | 5 | 14 |
| * | 3 | 3 | 2 | 21 |
| | 4 | 18 | 15 | 11,7 |

*$p < 0.05$ difference as compared to the control group according to Mann Whitney U Test

The results showed a significant increase in the number of penile erections and masturbations among treated animals when compared to controls. Evidently, there was no difference in the serum testosterone level between treated animals and controls, nor was there a correlation between these values and the number of erections and masturbations.

This experiment suggests that the administration of this formulation increases libido in treated animals, and that this effect is not related to the level of masculine hormones found in the group of monkeys.

Masculine sexual behaviour in mammals includes libido, penile erection, ejaculation and orgasm. This behaviour is determined by the release of testicular hormones acting on peripheral effector organs and provoking feedback effects in specific brain areas mainly located in the mediobasal hypothalamic region.

According to **Buffum J.** *et al*, **Handbook of Sexology, Vol. 6 The Pharmacology and Endocrinology of sex and function 462 (1988)**, libido (or sex desire) is mainly controlled by the limbic system through the dopaminergic and serotonergic pathways. Minimal levels of testosterone are necessary to maintain libido but, according to what the author himself stated in 1982, additional increases of testosterone do not lead to a more intense urge. Logically enough, penile erection is affected as such by libido, but this is also a process regulated by the autonomic nervous system.

Consequently, the effects caused by the administration of mixture of fatty alcohols may be a result of various mechanisms and are not directly deduced from the change in the hormone pattern. Yet, in an effort to determine how this mixture of primary fatty alcohols affects the sexual behaviour of male rats, there were experiments which included the quantifying of copulations in castrated rats with and without mixture and in the presence of an exogenic supply of testosterone, as well as in castrated rats with an without that alcohol mixture, but in the absence of an exogenic administration of testosterone. The following example is taken from the latter series.

**Example 10**

Thirty castrated rats were taken and divided into three identical groups. One group was taken as a control and the other two were given 1 and 5 mg/kg of fatty alcohol The of the pharmaceutical formulation. The results of these experiments are shown in Table XII.

TABLE XII

| Effects of the mixture of primary fatty alcohols on the sexual behavior of castrated rats | | | | |
|---|---|---|---|---|
| Volumes of alcohol mixture | Before castration | | After castration (under treatment) | |
| | Mount | Erection | Mount | Erection |
| | $(\overline{X} \pm DS)$ | | | |
| Control | $55 \pm 46$ | $55 \pm 45$ | $17 \pm 23+$ | $15 \pm 25+$ |
| 1 | $56 \pm 44$ | $56 \pm 44$ | $40 \pm 50$ | $40 \pm 50$ |
| 5 | $55 \pm 45$ | $55 \pm 44$ | $55 \pm 47$ | $51 \pm 47$ |

\*$p < 0.05$ with regards to control (Mann Whitney U Test)
+$p < 0.01$ with regards to previous observation (Wilcoxon)

This experiment showed that, in the absence of the main source of an exogenic supply of testosterone, there are still very significant differences between rats which were administered the fatty alcohol mixture and the controls, which might be attributed in principle to an effect on the testosterone threshold value required in the central nervous system for maintaining libido.

**Claims**

1.  Mixture of higher primary aliphatic alcohols, obtainable from sugar cane wax, ranging from 24 to 34 carbon atoms, characterized in that its composition is as follows:

| | |
|---|---|
| tetracosanol | 0.5 - 5.0 % |
| hexacosanol | 5.0 - 15.0 % |
| heptacosanol | 0.5 - **5.0 %** |
| octacosanol | 50.0 - 80.0 % |
| nonacosanol | 0.5 - 3.0 % |
| triacontanol | 6.0 - **20.0 %** |
| dotriacontanol | 1.0 - 10.0 % |
| tetratriacontanol | 0.0 - 2.5 % |

2.  Pharmaceutical formulation characterized by containing as an active compound the mixture of aliphatic alcohols ranging from 24 to 34 carbon atoms as decribed in claim 1, and agglutinants, fillers, disintegrators, lubricants and other pharmaceutically acceptable excipients used for tablets, capsules or granules.

3.  Pharmaceutical formulation according to claim 2, characterized by its content of the mixture of alcohols as an active compound in a 0.5 - 5.0 % proportion in respect to the total weight.

4.  Use of a mixture according to claim 1 for the preparation of a pharmaceutical formulation to be used as a hypocholesterolaemic.

5. Use of a mixture according to claim 1 for the preparation of a pharmaceutical formulation for the treatment of hyperlypoproteinaemia type II.

6. Use of a mixture according to claim 1 for the preparation of a pharmaceutical formulation for improving sexual activity.

**Patentansprüche**

1. Eine Mischung höherer, aliphatischer Primäralkohole, erhältlich aus Zuckerrohrwachs, mit 24 bis 34 Kohlenstoffatomen, dadurch gekennzeichnet, dass sie sich wie folgt zusammensetzt:

| | |
|---|---|
| Tetracosanol | 0,5 - 5,0 % |
| Hexaconsanol | 5,0 - 15,0 % |
| Heptacosanol | 0,5 - 5,0 % |
| Octacosanol | 50,0 - 80,0 % |
| Nonacosanol | 0,5 - 3,0 % |
| Triacontanol | 6,0 - 20,0 % |
| Dotriacontanol | 1,0 - 10,0 % |
| Tetratriacontanol | 0,0 - 2,5 % |

2. Ein Pharmaprodukt dadurch gekennzeichnet, dass es als aktive Verbindung die Mischung von aliphatischen Alkoholen mit 24 bis 34 Kohlenstoffatomen enthält, wie im Patentanspruch 1 beschrieben, und Bindemittel, Füllstoffe, Desintegriermittel, Schmiermittel und andere pharmazeutisch akzeptierbare Träger für die Verwendung in Tabletten, Kapseln oder Granulat.

3. Ein pharmazeutisches Produkt übereinstimmend mit Anspruch 2 dadurch gekennzeichnet, dass es als aktive Verbindung eine Alkoholmischung in einem Verhältnis von 0,5 - 5,0 % bezüglich des Gesamtgewichtes enthält.

4. Der Gebrauch einer Mischung gemäss Anspruch 1 für die Zubereitung eines pharmazeutischen Produktes für die Verwendung als Mittel gegen Hypocholesterinaemie.

5. Der Gebrauch einer Mischung gemäss Anspruch 1, für die Zubereitung eines pharmazeutischen Produktes für die Behandlung der Hyperlipoproteinaemie, Typ II.

6. Der Gebrauch einer Mischung gemäss Anspruch 1 für die Zubereitung eines pharmazeutischen Produktes zur Steigerung der sexuellen Aktivität.

**Revendications**

1. Mélange d'alcools aliphatiques primaires supérieurs pouvant être obtenu à partir de cire de canne à sucre, dont l'intervalle se situe entre 24 et 34 atomes de carbone, caractérisé en ce que sa composition est la suivante :

| | |
|---|---|
| tetracosanol | 0,5 - 5,0 % |
| hexacosanol | 5,0 - 15,0 % |
| heptacosanol | 0,5 - 5,0 % |
| octacosanol | 50,0 - 80,0 % |
| nonacosanol | 0,5 - 3,0 % |

(continued)

| triacontanol | 6,0 - 20,0 % |
|---|---|
| dotriacontanol | 1,0 - 10,0 % |
| tetratriacontanol | 0,0 - 2,5 % |

2. Formulation pharmaceutique caractérisée en ce qu'elle contient comme composé actif, le mélange d'alcools aliphatiques dont l'intervalle se situe entre 24 et 34 atomes de carbone comme le décrit la Revendication 1 et des agglutinants, des produits d'apport, des désintégrateurs, des lubrifiants et autres excipients pharmaceutiquement acceptables utilisés pour des comprimés, capsules ou granulés.

3. Formulation pharmaceutique selon la Revendication 2, caractérisée en ce qu'elle contient le mélange d'alcools comm composé actif dans une proportion de 0,5 - 5,0 % par rapport au poids total.

4. Utilisation d'un mélange selon la Revendication 1 pour la préparation d'une formulation pharmaceutique devant être employée comme hypocholestérolaémique.

5. Utilisation d'un mélange selon la Revendication 1 pour la préparation d'une formulation pharmaceutique pour le traitement de l'hyperlypoprotéinaémie type II.

6. Utilisation d'un mélange selon la Revendication 1 pour la préparation d'une formulation pharmaceutique pour améliorer l'activité sexuelle.